# EUROPEAN PATENT APPLICATION

(11) **EP 2 700 398 A1**
(43) Date of publication of application: **26.02.2014**
(21) Application number: 13180731.5
(22) Date of filing: 16.08.2013
(51) Int. Cl.: A61K 9/00, A61K 31/138, A61K 31/4985, A61K 9/46, A61K 9/16

(54) **Effervescent Sachet Formulations of Dapoxetine and a Pde5 Inhibitor**

(30) Priority: 17.08.2012 TR 201209599; 31.10.2012 TR 201212488; 07.11.2012 TR 201212850; 07.02.2013 TR 201301536; 08.02.2013 TR 201301559; 30.05.2013 TR 201306476; 25.07.2013 TR 201309037
(71) Applicant: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR)
(74) Representative: Sevinç, Erkan

(57) **Abstract**

The present invention relates to novel effervescent sachet formulations of dapoxetine or a pharmaceutically acceptable salt thereof and a phosphodiesterase type 5 inhibitor or a pharmaceutically acceptable salt thereof. The present invention further relates to a process for preparing such a formulation and to the use thereof in the treatment of erectile dysfunction.

## Description

### Technical Field

The present invention relates to a formulation comprising a combination of dapoxetine or a pharmaceutically acceptable salt thereof with a phosphodiesterase type 5 inhibitor. The present invention more particularly relates to an effervescent sachet formulation of dapoxetine and a phosphodiesterase type 5 inhibitor, which provides desired properties.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which was first disclosed in the European patent publication EP 0288188 B1 is a selective serotonin reuptake inhibitor. Dapoxetine is used for the treatment of depression and premature ejaculation and has the chemical structure shown in Formula I. Additionally, dapoxetine was approved in Switzerland and in Finland for use in the treatment of premature ejaculation.

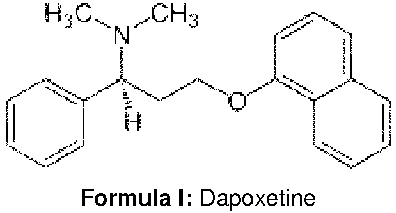

Following oral administration, dapoxetine is rapidly absorbed and rapidly enters the blood circulation by almost completely binding to plasma proteins. Therefore, it reaches the peak plasma concentration (Cmax) in 1 hour following oral administration. Orally-administered tablets of dapoxetine are commercially available under the name Priligy^{®}, comprising 30 mg or 60 mg dapoxetine hydrochloride as the active agent per tablet, as well as excipients including lactose monohydrate, microcrystalline cellulose, croscarmellose sodium, colloidal anhydrous silica, magnesium stearate, hypromellose, titanium dioxide (E171), triacetin, black iron oxide (E172) and yellow iron oxide.

The most frequently encountered problem in oral dapoxetine formulations is the bitter taste thereof. The tablets have typically been coated with coating agents; and mixtures of sweeteners or cation exchange resins have been used for masking the bitter taste.

On the other hand, phosphodiesterase type 5 inhibitors (PDE5 inhibitor) are used in the treatment of erectile dysfunction (ED). PDE5 inhibitors block the phosphodiesterase enzyme in a selective and efficient manner, thereby increasing the level of cyclic guanosine monophosphate (cGMP) in the corpus cavernosum smooth muscle cells. Most frequently used PDE5 inhibitors are avanafil, lodenafil, mirodenafil, sildenafil, tadalafil, vardenafil, and udenafil.

Avanafil, which is commercially available under the name Stendra^{®}, is a PDE5 inhibitor used in the treatment of ED. It is more rapidly effective than other PDE5 inhibitors. The chemical name of avanafil is (S)-4-[(3-chloro-4-methoxybenzyl)amino]-2-[2-(hydroxymethyl)-1-pyrrolidinyl]-N-(2-pyrrimidinylmethyl)-5-pyrrimidinecarboxyamide, with the chemical structure illustrated below in Formula II.

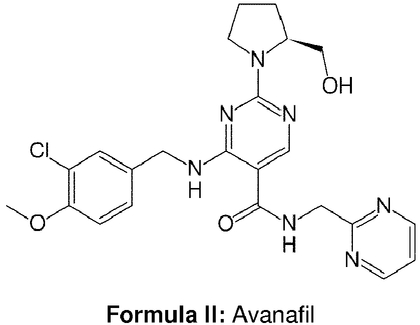

Lodenafil, which is commercially available under the name Helleva^{®}, is a new generation PDE5 inhibitor. It is formulated as a dimer and is converted into two active lodenafil molecules upon administration to the body. This enhances the bioavailability of the drug. The chemical name of lodenafil is bis-(2-{4-[4-ethoxy-3-(1-methyl-7-oxo-3-propyl-6,7-dihydro-1H-pyrazole[4,3-d]pyrimidine-5-yl)-benzenesulfonyl]piperazine-1-yl}-ethyl)carbonate, with the chemical structure illustrated below in Formula III.

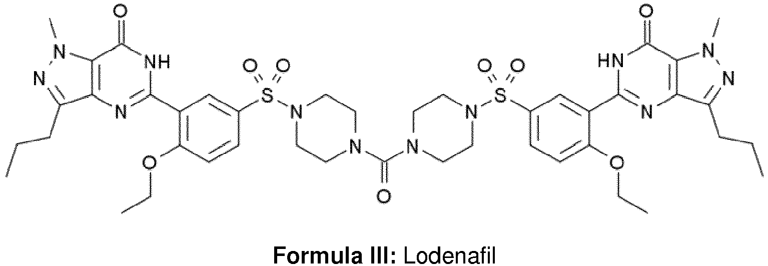

Mirodenafil, which is commercially available under the name Mvix^{®}, is a PDE5 inhibitor used in the treatment of ED. An orally-disintegrating film dosage form thereof is commercially available under the name Mvix S^{®}. The chemical name of mirodenafil is 5-ethyl-3,5-dihydro-2-[5-([4-(2-hydroxyethyl)-1-piperazinyl]sulfonyl)-2-propoxyphenyl]-7-propyl-4H-pyrrole[3,2-d]pyrimidine-4-one, with the chemical structure illustrated below in Formula IV.

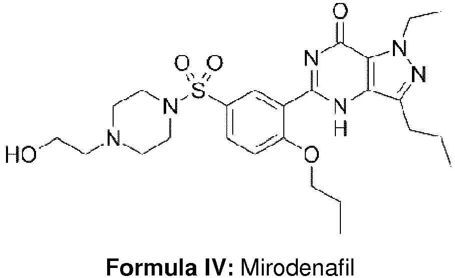

Sildenafil is a PDE5 inhibitor used in the treatment of ED and pulmonary artery hypertension (PAH). It is commercially available under the names Viagra^{®} and Revatio^{®}. The chemical name of sildenafil is 1-[4-ethoxy-3-(6,7-dihydro-1-methyl-7-oxo-3-propyl-1 H-pyrazole[4,3-d]pyrimidine-5-yl)phenylsulfonyl]-4-methylpiperazine, with the chemical structure illustrated below in Formula V.

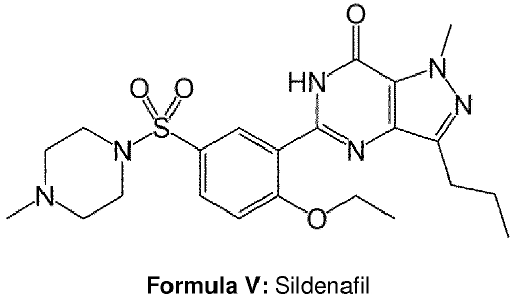

Tadalafil is a PDE5 inhibitor used in the treatment of ED and PAH. It has a longer half-life (average, 17,5 hours) as compared to other PDE5 inhibitors. The chemical name of tadalafil is (6R-trans)-6-(1,3-benzodioxol-5-yl)-2,3,6,7,12,12a-hexahydro-2-methyl-pyrazino[1',2':1,6]pyrido[3,4-b]indole-1,4-dione, with the chemical structure illustrated below in Formula VI.

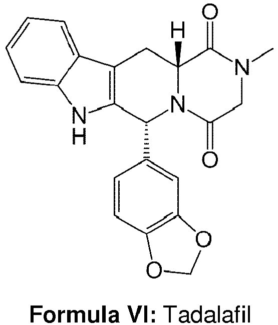

Vardenafil, which is commercially available under the name Levitra^{®}, is a PDE5 inhibitor used in the treatment of ED. It is further commercially available in an orally disintegrating tablet form, named Staxyn^{®}. The chemical name of vardenafil is 4-[2-ethoxy-5-(4-ethylpiperazine-1-yl)sulfonyl-phenyl]-9-methyl-7-propyl-3,5,6,8-tetrabicyclo[4.3.0]nona-3,7,9-triene-2-one, with the chemical structure illustrated below in Formula VII.

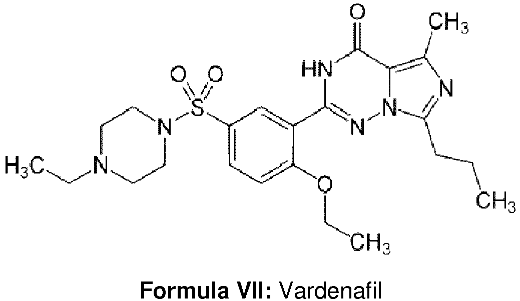

Udenafil, which is commercially available under the name Zydena^{®}, is a PDE5 inhibitor used in the treatment of ED. The chemical name of udenafil is 3-(1-methyl-7-oxo-3-propyl-4,7-dihydro-1H-pyrazole[4,3-d]pyrimidine-5-yl)-N-[2-(1-methylpyrrolidine-2-yl)ethyl]-4-propoxybenzenesulfonamide, with the chemical structure illustrated below in Formula VIII.

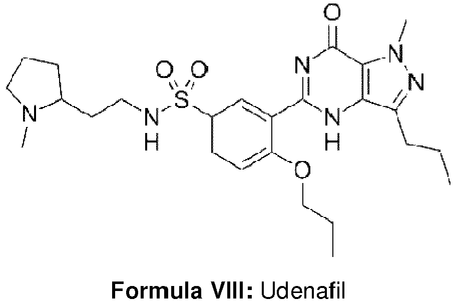

Formulations comprising a combination of selective serotonin reuptake inhibitors with PDE5 inhibitors are known in the prior art. The patent publication WO03000343, for example, discloses the use of a formulation comprising a combination of phosphodiesterase inhibitors and dapoxetine. On the other hand, MJ. Dresser et al. stated that dapoxetine, a selective serotonin reuptake inhibitor, does not have pharmaceutical interactions with PDE5 inhibitors and may be used for the treatment of premature ejaculation (Int J Impot Res. 2006 Jan-Feb;18(1):104-10). However, no effervescent sachet formulations are available which comprise dapoxetine and a PDE5 inhibitor.

The first aim of the effervescent sachet formulations comprising a combination of dapoxetine or a pharmaceutically acceptable salt thereof and a PDE5 inhibitor or a pharmaceutically acceptable salt thereof is to solve the problem resulting from the PDE5 inhibitor, for example to provide a high water-disintegration and water-solubility and therefore a high bioavailability for the effervescent sachet form. Effervescent sachet formulations with a good solubility result in a homogenous mixture, and the patient compliance is increased accordingly. It is known that PDE5 inhibitors are weakly soluble in water; this, in turn, leads to a low dissolution rate in aqueous media such as the gastrointestinal fluid, and consequently, a low bioavailability is observed following oral digestion. Various methods have been used to overcome this problem. For instance, although tadalafil is poorly soluble in water, it can dissolve in organic solvents such as dimethylformamide and dimethylsulfoxide. In the patent publication EP1200091 B1, hydrophilic solvents such as polyethylene glycol 400, propylene glycol, and glycofurol are used to dissolve tadalafil, which is weakly soluble in water. According to that patent, tadalafil is solved in hydrophilic solvents and then filled into soft capsules. US6821975 patent describes that tadalafil particles are micronized to overcome the solubility problem of tadalafil. The patent publication US20080009502, in turn, describes a solid composite comprising tadalafil and at least one hydrophilic carrier.

Various formulations and methods for preparing effervescent sachet formulations are already known. However, concerning oral administration, effervescent sachet formulations have become an issue with increasing importance in terms of patient compliance as compared to conventional solid dosage forms such as capsules and tablets. This issue is more important in terms of patients having difficulty in swallowing. For these reasons, effervescent sachet formulations are one of the advantageous routes for administering the drugs comprising dapoxetine and a PDE5 inhibitor and provide a higher patient compliance along with recommended pharmaceutical therapies. Developing an effervescent sachet composition is known to be difficult for several different reasons. Effervescent sachet formulations meet some requirements. First, leaving an unpleasant and bitter taste upon dissolution in water may cause problems. The homogenized of a dissolved effervescent sachet is an advantageous criterion in terms of patient compliance concerning the use of the medicament, and is thus preferable.

In order to fulfill all these requirements described above, a special drug formulation should be made by carefully selecting the excipients used in the preparation thereof. Thus, an improper selection of excipients may give formulations with a poor bioavailability as compared to equivalent conventional dosage forms. For this reason, the excipients should be selected very carefully.

Therefore, an improved pharmaceutical formulation is needed comprising dapoxetine and a PDE5 inhibitor, which overcomes the problems of the related prior art. Other advantages and embodiments of the present invention will be clarified in the following description.

### Detailed Description of Invention

The main object of the present invention is to provide an improved effervescent sachet formulation of a combination of dapoxetine or a pharmaceutically acceptable salt thereof and a PDE5 inhibitor or a pharmaceutically acceptable salt thereof by making use of appropriate excipients, which overcomes the problems mentioned above and is useful in the treatment of erectile dysfunction and the associated symptoms thereof.

A further object of the present invention is to eliminate the problems related to active ingredients and to obtain a solution with pleasant taste when the sachet formulation dissolves in water. Another object is to provide an effervescent sachet formulation, which has good water-solubility and is stable during shelf life.

With the present invention, an effervescent sachet formulation comprising dapoxetine and a PDE5 inhibitor is obtained, which has good solubility and dissolution rate, and thus high bioavailability and does not have a bitter taste.

According to another object of the present invention, defined amounts of acid and base sources are used to obtain a good water-soluble novel effervescent sachet, which comprises dapoxetine or a pharmaceutically acceptable salt thereof, a PDE5 inhibitor or a pharmaceutically salt thereof. Accordingly, it was observed that an acid to base ratio in the formulation ranging from 6:1 to 1:6, preferably from 4:1 to 1:4, more preferably from 2:1 to 1:2 increased the solubility of the formulation.

The acid source for use in the formulation according to the present invention is selected from a group comprising citric acid, citric acid monohydrate, citric acid anhydrate, fumaric acid, tartaric acid, ascorbic acid, malic acid, acetylsalicylic acid, sodium dihydrogen citrate, sodium citrate, sodium citrate anhydrate, disodium hydrogen citrate, nicotinic acid, adipic acid, or the mixtures thereof.

The base source for use in the formulation according to the present invention is selected from a group comprising sodium bicarbonate, sodium carbonate, sodium hydrogen carbonate, potassium bicarbonate, potassium carbonate, amino acid-alkali metal carbonate derivatives, or the mixtures thereof.

A further object of the present invention is to increase the patient compliance by providing sachet formulation without bitter taste. According to one embodiment, it was found that the selection of the sweetener from sucralose, thaumatin, mogroside, inuline, erythritol or a mixture thereof gave good results in masking the bitter taste resulting from dapoxetine. This is because sucralose, thaumatin, or mogroside is 300 to 3000 times more sweeter than sucrose. Using sucralose, thaumatin, or mogroside in low amounts both provides a pleasant taste for the effervescent sachet formulation and enhances the compliance of patients to the treatment. Additionally, since sucralose, thaumatin, mogroside, inuline, and erythritol are natural sweeteners, they are non-caloric, are not carcinogenic, and do not cause tooth decays and glycemic effect. By virtue of these natural sweeteners which are non-caloric, the effervescent formulations according to the present invention can also be used by diabetic patients. Additionally, the amount of the sweeteners is 0.001 to 15.00% by weight, preferably 0.01 to 10.00% by weight and more preferably 0.10 to 5.00% by weight in the effervescent sachet formulation and these amounts make it possible to significantly improve the taste of the formulation. It was unexpectedly found that in addition to the acid - base sources, using these sweeteners in the preferred amounts increases the solubility of the effervescent sachet formulation and so using these sweeteners in the preferred amounts has a synergic effect increasing the patient compliance.

According to another embodiment, the amount of dapoxetine or a pharmaceutically acceptable salt thereof is 5.00 to 85.00% by weight and the amount of a PDE5 inhibitor or a pharmaceutically acceptable salt thereof is 5.00 to 85.00% by weight in the effervescent sachet formulation.

The PDE5 inhibitor for use in the effervescent sachet formulation according to the present invention is selected from a group comprising avanafil, lodenafil, mirodenafil, sildenafil, tadalafil, vardenafil, and udenafil; and the preferred PDE5 inhibitor is tadalafil.

The onset of action time and the action time of each PDE5 inhibitor are different. Following oral administration, the onset of action of sildenafil and tadalafil take 1 hour and 15 minutes, respectively. The onset of action of sildenafil takes 4 hours, and that of tadalafil may take up to 36 hours. Tadalafil is preferred for embodiments in which a rapid action onset is desired.

In addition to the active agents, sweeteners, and the acid and base sources, the effervescent sachet formulation according to the present invention further comprises at least one pharmaceutically acceptable excipient selected from the group comprising of disintegrants, binders, fillers, flavoring agents and preferably antioxidants.

Suitable disintegrants for use in the formulations according to the present invention include, but are not limited to alginic acid and alginates, ion-exchange resins, magnesium aluminum silicate, sodium dodecyl sulfate, sodium carboxymethyl cellulose, croscarmellose sodium, crospovidone, carboxymethyl cellulose calcium, docusate sodium, guar gum, low-substituted hydroxypropyl cellulose, polyacrylin potassium, poloxamer, sodium alginate, sodium glycine carbonate, sodium lauryl sulfate, or the mixtures thereof. The amount of the disintegrants is 1.00 to 40.00 % by weight, preferably 1.00 to 30.00 % by weight in the effervescent sachet formulation.

Suitable binders for use in the formulations according to the present invention include, but are not limited to sugars, glucose syrup, natural gums, starch, gelatin, polyvinylpyrrolidone, vinylpyrrolidone-vinyl acetate copolymer, polymethacrylates; collagen, gelatin, agar, alginate, sodium alginate, pectin, carboxymethyl cellulose, hydroxypropyl cellulose, methyl cellulose, carbomer, poloxamer, polyacrylamide, polyvinyl alcohol, aluminum hydroxide, bentonite, laponite, starch mucilage, acacia mucilage, polydextrose, polyethylene oxide, or the mixtures thereof. The amount of the binders is 0.10 to 40.00 % by weight, preferably 1.00 to 30.00 % by weight in the effervescent sachet formulation.

Suitable fillers for use in the formulations according to the present invention include, but are not limited to mannitol, sorbitol, sucrose, inorganic salts, calcium salts, polisaccharides, dextrose, trehalose, lactitol, xylitol, maltodextrin, or the mixtures thereof. The amount of the fillers is 1.00 to 90.00 % by weight, preferably 5.00 to 90.00 % by weight in the effervescent sachet formulation.

Suitable flavoring agents for use in the formulations according to the present invention include, but are not limited to fruit aromas such as orange, banana, strawberry, cherry, wild cherry, lemon, and other aromas such as cardamom, anis, mint, menthol, vanillin, ethyl vanillin or the mixtures thereof. The amount of the flavoring agent is 0.10 to 10.00 % by weight, preferably 0.10 to 5.00 % by weight in the effervescent sachet formulation.

Suitable antioxidants for use in the formulations according to the present invention include, but are not limited to butyl hydroxyanisole, butyl hydroxytoluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, alpha tocopherol, or the mixtures thereof.

The present invention is described in the following examples in more details. These examples are not limiting the scope of the present invention and should be considered under the light of the foregoing detailed disclosure.

### Examples

### Example 1: Effervescent sachet comprising dapoxetine and tadalafil

| **Ingredients** | **Amount (%)** |
|---|---|
| Tadalafil | 5.00 - 85.00 |
| Dapoxetine | 5.00 - 85.00 |
| Sodium bicarbonate | 5.00 - 60.00 |
| Sodium citrate anhydrate | 5.00 - 60.00 |
| Citric acid anhydrate | 5.00 - 60.00 |
| Tartaric acid | 5.00 - 60.00 |
| Sucralose | 0.10 - 0.20 |
| Thaumatin | 0.10 - 0.20 |
| Flavor | 0.10 - 5.00 |

### Production method:

Tadalafil, dapoxetine, sodium bicarbonate, citric acid anhydrate, sodium citrate anhydrate, tartaric acid, sucralose, and the flavor are passed through a 1.00 mm sieve and mixed until a homogeneous mixture is obtained. The resulting mixture is filled into appropriate sachet package.

### Example 2: Effervescent sachet comprising dapoxetine and sildenafil

| **Ingredients** | **Amount (%)** |
|---|---|
| Sildenafil | 5.00 - 85.00 |
| Dapoxetine | 5.00 - 85.00 |
| Sodium bicarbonate | 5.00 - 60.00 |
| Citric acid anhydrate | 5.00 - 60.00 |
| Sodium citrate anhydrate | 5.00 - 60.00 |
| Tartaric acid | 5.00 - 60.00 |
| Sucralose | 0.10 - 0.20 |
| Thaumatin | 0.10 - 0.20 |
| Flavor | 0.10 - 5.00 |

### Production method:

Sildenafil, dapoxetine, citric acid anhydrate, tartaric acid, sodium bicarbonate and sodium citrate anhydrate are sieved and mixed. The mixture is dried at 105°C in a drying oven. Dried granules are passed through a 1.00 mm sieve. Sucralose, thaumatin and the flavor are added to the dried granules and stirred until a homogenous mixture is obtained. The resulting mixture is filled into appropriate sachet package.

### Example 3: Effervescent sachet comprising dapoxetine and vardenafil

| **Ingredients** | **Amount (%)** |
|---|---|
| Dapoxetine | 5.00 - 85.00 |
| Vardenafil | 5.00 - 85.00 |
| Malic acid | 5.00 - 60.00 |
| Sodium bicarbonate | 5.00 - 60.00 |
| Sucralose | 0.01 - 2.00 |
| *Kollidon^{®} CL* (crospovidone) | 1.00 - 30.00 |
| *Kollidon^{®} VA* 64 (vinylpyrrolidone-vinyl acetate copolymer) | 1.00 - 30.00 |
| Flavor | 0.10 - 5.00 |

### Production method:

*Kollidon^{®} VA* 64 solution is prepared with water or alcohol - water mixture. Vardenafil, dapoxetine, malic acid, sodium bicarbonate and *Kollidon^{®} CL* are mixed and this powder mixture is granulated with prepared solution. Then, the mixture is dried in a drying oven. Dried granules are passed through a 1.00 mm sieve. Sucralose and the flavor are added to the dried granules and stirred until a homogenous mixture is obtained. The resulting mixture is filled into appropriate sachet package.

### Example 4: Effervescent sachet comprising dapoxetine and avanafil

| **Ingredients** | **Amount (%)** |
|---|---|
| Dapoxetine | 5.00 - 85.00 |
| Avanafil | 5.00 - 85.00 |
| Malic acid | 5.00 - 60.00 |
| Sodium bicarbonate | 5.00 - 60.00 |
| Sucralose | 0.01 - 2.00 |
| *Kollidon^{®} CL* (crospovidone) | 1.00 - 30.00 |
| *Kollidon^{®} VA 64* (vinylpyrrolidone-vinyl acetate copolymer) | 1.00 - 30.00 |
| Flavor | 0.10 - 5.00 |

### Production method:

*Kollidon^{®} VA 64* solution is prepared with alcohol or alcohol - water mixture. Avanafil, dapoxetine, malic acid, sodium bicarbonate and *Kollidon^{®} CL* are mixed and this powder mixture is granulated with prepared solution. Then, the mixture is dried in a drying oven. Dried granules are passed through a 1.00 mm sieve. Sucralose and the flavor are added to the dried granules and stirred until a homogenous mixture is obtained. The resulting mixture is filled into appropriate sachet package.

### Example 5: Effervescent sachet comprising dapoxetine and lodenafil

| **Ingredients** | **Amount (%)** |
|---|---|
| Dapoxetine | 5.00 - 85.00 |
| Lodenafil | 5.00 - 85.00 |
| Trehalose | 5.00 - 90.00 |
| Tartaric acid | 5.00 - 60.00 |
| Sodium hydrogen carbonate | 5.00 - 60.00 |
| Sodium citrate | 5.00 - 60.00 |
| Polyvinylpyrrolidone | 0.10 - 25.00 |
| Sucralose | 0.10 - 2.00 |
| Mogroside | 0.10 - 2.00 |
| Flavor | 0.10 - 5.00 |

### Production method:

Polyvinylpyrrolidone solution is prepared with alcohol or alcohol - water mixture. Lodenafil and dapoxetine are mixed with trehalose. This powder mixture is granulated with prepared solution. Then, the mixture is dried in a drying oven. Dried granules are passed through a 1.00 mm sieve. Tartaric acid, sodium hydrogen carbonate, sodium citrate, sucralose, mogroside and the flavor are added to the dried granules and stirred until a homogenous mixture is obtained. The resulting mixture is filled into appropriate sachet package.

### Example 6: Effervescent sachet comprising dapoxetine and mirodenafil

| **Ingredients** | **Amount (%)** |
|---|---|
| Dapoxetine | 5.00 - 85.00 |
| Mirodenafil | 5.00 - 85.00 |
| Sodium bicarbonate | 5.00 - 60.00 |
| Citric acid anhydrate | 5.00 - 60.00 |
| Sodium citrate anhydrate | 5.00 - 60.00 |
| Tartaric acid | 5.00 - 60.00 |
| Sucralose | 5.00 - 60.00 |
| Thaumatin | 0.10 - 2.00 |
| Flavor | 0.10 - 5.00 |

### Production method:

Mirodenafil, dapoxetine, citric acid anhydrate, tartaric acid, sodium bicarbonate and sodium citrate anhydrate are sieved and mixed. The mixture is dried at 105°C in a drying oven. Dried granules are passed through a 1.00 mm sieve. Sucralose, thaumatin and the flavor are added to the dried granules and stirred until a homogenous mixture is obtained. The resulting mixture is filled into appropriate sachet package.

### Example 7: Effervescent sachet comprising dapoxetine and tadalafil

| **Ingredients** | **Amount (%)** |
|---|---|
| Dapoxetine | 5.00 - 85.00 |
| Tadalafil | 5.00 - 85.00 |
| Malic acid | 5.00 - 60.00 |
| Sodium bicarbonate | 5.00 - 60.00 |
| Sucralose | 0.10 - 2.00 |
| *Kollidon^{®} CL* (crospovidone) | 1.00 - 30.00 |
| *Kollidon^{®} VA 64* (vinylpyrrolidone-vinyl acetate copolymer) | 1.00 - 30.00 |
| Flavor | 0.10 - 5.00 |

### Production method:

*Kollidon^{®} VA 64* solution is prepared with alcohol or alcohol - water mixture. Tadalafil, dapoxetine, malic acid, sodium bicarbonate and *Kollidon^{®} CL* are mixed and this powder mixture is granulated with prepared solution. Then, the mixture is dried in a drying oven. Dried granules are passed through a 1.00 mm sieve. Sucralose and the flavor are added to the dried granules and stirred until a homogenous mixture is obtained. The resulting mixture is filled into appropriate sachet package.

### Example 8: Effervescent sachet comprising dapoxetine and mirodenafil

| **Ingredients** | **Amount (%)** |
|---|---|
| Dapoxetine | 5.00 - 85.00 |
| Mirodenafil | 5.00 - 85.00 |
| Sodium bicarbonate | 5.00 - 60.00 |
| Citric acid anhydrate | 5.00 - 60.00 |
| Sodium citrate anhydrate | 5.00 - 60.00 |
| Tartaric acid | 5.00 - 60.00 |
| Sucralose | 5.00 - 60.00 |
| Thaumatin | 0.10 -2.00 |
| Flavor | 0.10 - 5.00 |

### Production method:

Mirodenafil, dapoxetine, sodium bicarbonate, citric acid anhydrate, sodium citrate anhydrate, tartaric acid, sucralose, and the flavor are passed through a 1.00 mm sieve and mixed until a homogeneous mixture is obtained. The resulting mixture is filled into appropriate sachet package.

### Example 9: Effervescent sachet comprising dapoxetine and tadalafil

| **Ingredients** | **Amount (%)** |
|---|---|
| Dapoxetine | 5.00 - 85.00 |
| Tadalafil | 5.00 - 85.00 |
| Trehalose | 5.00 - 90.00 |
| Tartaric acid | 5.00 - 60.00 |
| Sodium hydrogen carbonate | 5.00 - 60.00 |
| Sodium citrate | 5.00 - 60.00 |
| Polyvinylpyrrolidone | 0.10 - 25.00 |
| Sucralose | 0.10 - 2.00 |
| Mogroside | 0.10 - 2.00 |
| Flavor | 0.10 - 5.00 |

### Production method:

Polyvinylpyrrolidone solution is prepared with alcohol or alcohol - water mixture. Tadalafil and dapoxetine are mixed with trehalose. This powder mixture is granulated with prepared solution. Then, the mixture is dried in a drying oven. Dried granules are passed through a 1.00 mm sieve. Tartaric acid, sodium hydrogen carbonate, sodium citrate, sucralose, mogroside and the flavor are added to the dried granules and stirred until a homogenous mixture is obtained. The resulting mixture is filled into appropriate sachet package.

### Example 10: Effervescent sachet comprising dapoxetine and tadalafil

| **Ingredients** | **Amount (%)** |
|---|---|
| Tadalafil | 5.00 - 85.00 |
| Dapoxetine | 5.00 - 85.00 |
| Sodium bicarbonate | 5.00 - 60.00 |
| Sodium citrate anhydrate | 5.00 - 60.00 |
| Citric acid anhydrate | 5.00 - 60.00 |
| Tartaric acid | 5.00 - 60.00 |
| Sucralose | 0.10 - 0.20 |
| Thaumatin | 0.10 - 0.20 |
| Flavor | 0.10 - 5.00 |

### Production method:

Tadalafil, dapoxetine, citric acid anhydrate, tartaric acid, sodium bicarbonate and sodium citrate anhydrate are sieved and mixed. The mixture is dried at 105°C in a drying oven. Dried granules are passed through a 1.00 mm sieve. Sucralose, thaumatin and the flavor are added to the dried granules and stirred until a homogenous mixture is obtained. The resulting mixture is filled into appropriate sachet package.

## Claims

1. An oral effervescent sachet formulation comprising dapoxetine or a pharmaceutically acceptable salt thereof and a PDE5 inhibitor or a pharmaceutically acceptable salt thereof.

2. The effervescent sachet formulation according to claim 1, comprising at least one acid source and at least one base source.

3. The effervescent sachet formulation according to claim 2, wherein the ratio by weight of the acid source to the base source is between 6:1 and 1:6.

4. The effervescent sachet formulation according to claim 2, wherein the ratio by weight of the acid source to the base source is between 4:1 and 1:4.

5. The effervescent sachet formulation according to claim 2, wherein the ratio by weight of the acid source to the base source is between 2:1 and 1:2.

6. The effervescent sachet formulation according to claims 2-5, wherein the acid source is selected from a group comprising citric acid, citric acid monohydrate, citric acid anhydrate, fumaric acid, tartaric acid, ascorbic acid, malic acid, acetylsalicylic acid, sodium dihydrogen citrate, sodium citrate, sodium citrate anhydrate, disodium hydrogen citrate, nicotinic acid, adipic acid, or the mixtures thereof.

7. The effervescent sachet formulation according to claims 2-5, wherein the base source is selected from a group comprising sodium bicarbonate, sodium carbonate, sodium hydrogen carbonate, potassium bicarbonate, potassium carbonate, amino acid-alkali metal carbonate derivatives, or the mixtures thereof.

8. The effervescent sachet formulation according to claim 1, further comprising a sweetener or a mixture of sweeteners in an amount of 0.001 to 15.0% by weight based on the total weight of the effervescent sachet formulation.

9. The effervescent sachet formulation according to claim 1, wherein the amount of the sweetener is 0.01 to 10.00% by weight.

10. The effervescent sachet formulation according to claim 1, wherein the amount of the sweetener is 0.1 to 5.00% by weight.

11. The effervescent sachet formulation according to claims 8-10, wherein the sweetener is selected from a group comprising sucralose, thaumatin, mogroside, inuline, erythritol, or mixtures thereof.

12. The effervescent sachet formulation according to claim 11, wherein the sweetener is selected from a group comprising sucralose, thaumatin, mogroside, or mixtures thereof.

13. The effervescent sachet formulation according to claim 1, wherein the amount of dapoxetine or a pharmaceutically acceptable salt thereof is 5.0 to 85.0% by weight and the amount of PDE5 inhibitor or a pharmaceutically acceptable salt thereof is 5.0 to 85.0% by weight.

14. The effervescent sachet formulation according to claim 13, wherein the PDE5 inhibitor is selected from a group comprising avanafil, lodenafil, mirodenafil, sildenafil, tadalafil, vardenafil, and udenafil.

15. The effervescent sachet formulation according to claim 14, wherein the PDE5 inhibitor is tadalafil.
